# EUROPÄISCHE PATENTSCHRIFT

(11) **EP 1 940 341 B1**
(45) Veröffentlichungstag und Bekanntmachung des Hinweises auf die Patenterteilung: **05.08.2009**
(21) Anmeldenummer: 06806806.3
(22) Anmeldetag: 25.09.2006
(51) Int. Cl.: A61K 6/027, C03C 3/083, C03C 3/093

(54) **DENTALGLAS**
DENTAL GLASS
VERRE DENTAIRE

(30) Priorität: 27.10.2005 DE 102005051387
(43) Veröffentlichungstag der Anmeldung: 09.07.2008
(73) Patentinhaber: Ivoclar Vivadent AG, 9494 Schaan (LI); EMPA Dübendorf, 8600 Dübendorf (CH)
(72) Erfinder: RITZBERGER (GEB. VAN T'HOEN), Christian, A-6710 Nenzing (AT); RHEINBERGER, Volker, 9490 Vaduz (LI); APEL, Elke, CH-9475 Sevelen (CH); BURTSCHER, Peter, A-6830 Rankweil (AT); HÖLAND, Wolfram, 9494 Schaan (LI); GRAULE, Thomas, CH-8200 Schaffhausen (CH); ZÜRCHER, Simone, CH-6500 Bellinzona (CH); VITAL, Andri, CH-8032 Zürich (CH)
(74) Vertreter: Fitzner, Uwe
(86) Internationale Anmeldenummer: PCT/EP2006/066702
(87) Internationale Veröffentlichungsnummer: WO 2007/048670

(56) Entgegenhaltungen:
- DE-A1- 3 421 155

## Beschreibung

Die vorliegende Erfindung betrifft ein Dentalglas, ein Verfahren zu dessen Herstellung sowie dessen Verwendung.

Die heute verwendeten Dentalgläser sind überwiegend niedrigschmelzende Gläser mit einem dem Monomer bzw. der Monomermischung angepassten, niedrigen Brechungsindex von n=1,50 bis 1,53. Die bisher verwendeten Dentalgläser mit diesem niedrigen Brechungsindex besitzen jedoch den Nachteil einer geringen chemischen Beständigkeit, die sich im Anlösen oder gar Auflösen der Füllerkomponente im Komposit äußert.

Ein weit verbreitetes Dentalglassystem ist in der DE 43 23 143 C1 beschrieben. Dieses System SiO₂-BaO/SrO-B₂O₃ - Al₂O₃ - weist einen großen Anteil an SrO auf. Ein ähnliches System ist aus der US 6,121,344 bekannt, das BaO enthält.

Aus der DE 198 49 388 A1 sind ferner alkalioxidhaltige Gläser und deren Verwendung als Dentalfüller bekannt. Nachteilig ist bei diesem System, dass die Alkaliionen und das Fluor die chemische Beständigkeit des Glases herabsetzt.

In dem Stand der Technik werden WO₃ und La₂O₃-haltige Gläser beispielsweise im Bereich der Hochleistungsoptik verwendet (Glaschemie, S. 444ff, Vogel et al., Springer Verlag 1992). Diese Gläser besitzen aber einen hohen Brechungsindex. Ferner weisen sie sehr geringe SiO₂- und Phosphatgehalte auf. Im Hinblick auf die Brechzahl dieser Systeme sind derartige Gläser für Dentalanwendungen ungeeignet.

Aus der DE 34 21 155 A1 sind ferner Dentalgläser bekannt, die als Füllstoffkomponenten eingesetzt werden. Die dort beschriebenen Zusammensetzungen enthalten jedoch neben WO₃ und La₂O₃ sehr hohe Anteile an SrO. Der Brechungsindex dieses Glases liegt weiterhin bei 1,55 und höher. Aufgrund dieses hohen Brechungsindex und der anderen genannten Nachteile ist dieses Glas für den Einsatz als Dentalkomposit weniger geeignet.

Aus der US 6,121,344 ist schließlich ein Bariumsilikatglas bekannt, welches durch einen speziellen Mahlprozess hergestellt wird. Bei diesem Aufmahlprozess wird eine mittlere Korngröße von 500 nm (= 0,5 µm) erhalten. Da in den letzten Jahren verstärkt die Tendenz beobachtet wird, auf BaO wegen möglicher toxischer Wirkungen in Dentalgläsern zu verzichten, wird auf eine Verwendung dieser Gläser als Füllstoff für den Einsatz in der Dentaltechnik weitgehend verzichtet.

Aufgabe der Erfindung ist es nun, Gläser als anorganischen Füllstoff für Dentalkomposite bereitzustellen, die die bekannten Nachteile des Standes der Technik nicht mehr besitzen, insbesondere eine hohe Röntgenopazität und einen geeigneten niedrigen Brechungsindex aufweisen. Ebenso wird auf die Verwendung von Bariumoxid, Strontiumoxid und Alkalioxide verzichtet. Darüber hinaus sollen die mit Füllstoffen auf der Basis der erfindungsgemäßen Gläser hergestellten Dentalkomposite sehr gute mechanische Eigenschaften (Festigkeit und E-Modul), verbesserte Abrasionseigenschaften und gute optische Eigenschaften aufweisen. Darüber hinaus sollen die Brechungsindizes optimiert werden. Schließlich soll eine geeignete Röntgenopazität erreicht werden. Weiterhin sollen die Gläser auch eine gute chemische Beständigkeit gegenüber verdünnten NaF-Lösungen besitzen, da es infolge der Fluorid-Einwirkung auf silicatische Gläser zur Auslaugung oder sogar zum vollständigen Herauslösen der Füllstoffpartikel aus der ausgehärteten Monomermatrix des Composites kommen kann.

Aus der DE 197 13 048 A1 sind schließlich dentale Komposite auf der Basis von übl i-chen polymerisierbaren Monomeren, Initiatoren, Füllstoffen und anderen Hilfsstoffen, die als Schlack inhibierenden Zusatzsalze von säurefunktionellen Polymeren mit ein-oder mehrwertigen Kationen, vorzugsweise Glasionomerzemente enthalten. Aus der Offenbarung dieser Patentanmeldung geht aber kein Hinweis hervor, beispielsweise SrO und Alkalien zu vermeiden oder MgO und WO₃ aus einer ganzen Reihe gleichberechtigt genannter Bestandteile auszuwählen.

Die Aufgabe der Erfindung wird durch Dentalgläser umfassend 50 bis 70 Ma.-% SiO₂, 5 bis 18 Ma.-% Al₂O₃, 6,1 bis 30 Ma.-% MgO, 1 bis 15 Ma.-% La₂O₃, 1 bis 15 Ma.-% WO₃, 0,1 bis 8 Ma.-% ZrO₂ gelöst.

Die Summen von La₂O₃ + WO₃ betragen 8 bis 20 Ma.-% und von Al₂O₃+ La₂O₃ + WO₃ vorzugsweise 16 bis 38 Ma.-%. Die angegebenen Ma.-% addieren sich insgesamt auf 100 Ma.-%.
In einer bevorzugten Ausführungsform umfassen die Dentalgläser 50 bis 70 Ma.-% SiO₂, 9 bis 15 Ma.-% Al₂O₃, 6,1 bis 30 Ma.-% MgO, 1 bis 12 Ma.-% WO₃ und 0,1 bis 8 Ma.-% ZrO₃, enthält. Die Summe für La₂O₃ + Y₂O₃ beträgt vorzugsweise 1 bis 18 Ma.-%, die Summe für La₂O₃ + Al₂O₃ + Y₂O₃ + ZrO₂ beträgt vorzugsweise 16 bis 35 Ma.-%. Hierbei wird Y₂O₃ bevorzugt als teilweisen Ersatz für La₂O₃ verwendet.
In einer bevorzugten Ausführungsform enthalten die Dentalgläser 6,1 bis 20 Ma%MgO, vorzugsweise 6,1 bis 12 Ma%MgO.
Bevorzugt ist es ferner, dass die Dentalgläser 0 bis 15 Ma.-% Oxide von weiteren zweiwertigen Metallen, ausgenommen Sr und Ba, vorzugsweise CaO und ZnO enthalten.
Neben den Oxiden zweiwertiger Metalle können auch Oxide drei- oder vierwertiger Metalle in den Dentalgläsern enthalten sein. Bevorzugt sind Anteile von 0 bis 20 Ma.-% Oxide von dreiwertigen und/oder vierwertigen Metallen.
Zu den bevorzugten Oxiden gehören hier B₂O₃ sowie TiO₂.
Erfindungsgemäß ist es auch vorteilhaft, Ag₂O einzusetzen. Bevorzugt sind hier Mengen von 0 bis 4 Ma.-%, vorzugsweise 0.1 - 2.0 Masse%.

Gegebenenfalls können weitere Oxide mit Ausnahme der Oxide von Sr, Ba und der Alkalimetalle enthalten sein. Schließlich können in den erfindungsgemäßen Dentalgläsern noch Halogenide und Sulfate enthalten sein. Diese sind vorzugsweise in Mengen von 0 bis 2 Ma% enthalten.

Die gute chemische Beständigkeit, insbesondere gegenüber Fluorid-Einwirkungen, wird erreicht, indem die Verwendung solcher Ionen, die die Löslichkeit des Glases stark erhöhen, vermieden wird. Dies trifft insbesondere auf alle Alkaliionen zu. Für ein beständiges, schwer lösliches silicatisches Netzwerk werden deshalb vor allem Al₂O₃, ZrO₂, WO₃, La₂O₃ und Y₂O₃ eingebracht. Die Ionen des Ca, Mg und Zn ermöglichen eine bessere Schmelzbarkeit der Gläser und ermöglichen gleichzeitig eine gute und homogene Glasphase. Es wurde darüber hinaus überraschend gefunden, dass trotz der hohen Gehalte an WO₃ und La₂O₃ oder auch MgO keine Trübungserscheinungen festgestellt werden konnten. In einem solchen Fall wäre dieses Glas als Füllstoff von Dentalmaterialien nicht geeignet.
Überraschend gelang es, ein Magnesium-Aluminosilicat-Glas zu entwickeln, das hohe Gehalte von R(III)₂O₃ ( wobei R(III)₂O₃ die Summe aus La₂O₃ und Y₂O₃ darstellt), sowie WO₃ und ZrO₂ aufweist und eine hohe Röntgenopazität und einen Brechungsindex von nur etwa 1,50 bis 1,549 besitzt.

Die erfindungsgemäßen Zusammensetzungen haben weiterhin den überraschenden Effekt, dass einerseits eine hohe Röntgenopazität und andererseits ein Brechungsindex von 1,50 bis 1,549 erreicht werden. Abgesehen davon besitzen die mit den erfindungsgemäßen Dentalgläsern hergestellten Dentalmaterialien eine sehr gute chemische Beständigkeit. Da kein BaO und kein SrO oder Alkalioxide eingesetzt wurden, weist das Dentalglas bzw. der daraus hergestellte Füllstoff auch nicht die aus dem Stand der Technik bekannten Nachteile auf. Die genannten Brechungsindizes von 1,50 bis 1,549 wurden erreicht, obwohl insbesondere La₂O₃, Y₂O₃, WO₃ und ZrO₂ als Glaskomponenten zur Anhebung der Brechzahl von Gläsern verwendet wurden.

Die üblichen Monomere für Dentalkomposite besitzen einen Brechungsindex von etwa 1,47 bis 1,53. Höher brechende Monomere erfordern im Brechungsindex entsprechend angepasste Dentalgläser. Mit der erfindungsgemäßen Dentalglasentwicklung sind nunmehr Brechungsindizes im Bereich 1,50 bis 1,549 möglich geworden. Die erfindungsgemäßen Dentalgläser und die aus diesen Füllstoffen hergestellten Dentalmaterialien weisen eine gute chemische Beständigkeit gegenüber Fluoridlösungen auf, welche insbesondere in den Mundraum bei Anwendung entsprechender Zahnpasten oder Mundspüllösungen gelangen. Bei herkömmlichen Dentalgläsern kann es infolge von Fluorideinwirkungen zur Auslaugung oder zum vollständigen Herauslösen der Füllstoffpartikel aus der ausgehärteten Monomermatrix kommen. Dieser Prozeß führt zu Glanzverlust der Restauration, zunehmender Rauhigkeit sowie Belagbildung und stellt für den Patienten ein ästhetisches und auch hygienisches Problem dar.

Weiterer Vorteil der erfindungsgemäßen Dentalgläser ist, dass ihre Röntgenopazität verbessert ist. Dies ist für den Zahnarzt zur Erkennung von Überschüssen, Randspalten und Sekundärkaries wichtig. Dies wird wie bereits beschrieben durch die erfindungsgemäße Verwendung der Oxide des Zr, La, Y und W erreicht.

Die erfindungsgemäßen Dentalgläser werden für Füllstoffe grundsätzlich in Pulverform eingesetzt.
Ein weiterer Aspekt der vorliegenden Erfindung ist das Verfahren zur Herstellung der erfindungsgemäßen Dentalgläser als Pulver, bei dem
• eine Schmelze eines Ausgangsglases, welches die beschriebenen Oxide enthält, bei Temperaturen von ca. 1400°C bis ca. 1700°C, vorzugsweise bei 1600°C bis 1670°C erschmolzen und während einer Zeit von 0,5 bis 6 Stunden, vorzugsweise von 1 bis 2 Stunden homogenisiert wird,
• die Schmelze durch Fritten (schnelles Abkühlen der Schmelze durch Giessen in kaltes Wasser) abgeschreckt wird (Vermeidung der Phasentrennung und Eintrübung, zu der stark MgO-haltige Gläser gewöhnlich neigen) und Erzeugung von kleinen Glaskörnern.

Die auf dieser Art hergestellten Glaskörner werden anschließend vorzerkleinert. Hierbei wird auf eine mittlere Korngröße von 1 bis 50 µm, vorzugsweise 1 bis 20 µm, zerkleinert. Die Zerkleinerung erfolgt vorzugsweise in einer Strahl- oder Kugelmühle.

An die Vorzerkleinerung schließt sich eine Feinmahlung an. Hierbei erfolgt eine Zerkleinerung auf vorzugsweise 0,5 bis 5 µm, besonders bevorzugt 1 bis 2 µm. In einer bevorzugten Ausführungsform wird mittels Nassvermahlung gearbeitet. Hierfür werden vorzugsweise polyurethan- oder keramikausgekleidete Ringspalt-Kugelmühlen eingesetzt.

Die Nassvermahlung erfolgt unter Einsatz von Dispergiermitteln, vorzugsweise deionisiertem Wasser. Der Feststoffgehalt liegt bevorzugt bei 5 bis 50 Gew.-%, besonders bevorzugt zwischen 5 und 35 Gew.-%.

In einer bevorzugten Ausführungsform werden yttriumstabilisierte ZrO₂-Mahlkörper eingesetzt. Ebenso sind aber auch Al₂O₃- oder auch Glasmahlkörper der gleichen Zusammensetzung wie das erfindungsgemäße zur Feinmahlung geeignet.

Erfindungsgemäß wurde überraschenderweise gefunden, dass bei den angegebenen Feststoffgehalten eine zweistufige Feinstmahlung besonders vorteilhaft ist. Hierbei wird in einem ersten Verkleinerungsschritt mit einem Feststoffgehalt von 30 bis 40 Gew.-%, besonders bevorzugt ca. 33 Gew.-% und einem anschließendem zweiten Mahlschritt mit einem Feststoffgehalt von 5 bis 15 %, besonders bevorzugt von 10 Gew.-% die spezifische Oberfläche des Mahlgutes erheblich verändert. Nach etwa 40 Minuten Mahldauer bei 30 bis 40 Gew.-% Feststoffgehalt wird zunächst eine spezifische Oberfläche von 50 bis 60 m²/g und nach einer Verringerung des Feststoffgehaltes auf 5 bis 15 Gew.-% und weiteren 480 Minuten Mahldauer eine spezifische Oberfläche von 100 bis 110 m²/g erreicht.

Die spezifische Oberfläche kann mittels BET-Analyse (Beckman-Coulter, SA 3100) bestimmt. Die Suspension wurde dafür 30 min. bei 160°C getrocknet und 3 Stunden unter Stickstoff-Atmosphäre entgast. Die Bildauswertung einer Transmissions Elektronen Mikroskopischen-Aufnahme (Philips CM 30, Royal Philips Electronics, Eindhoven, NL) ergab, dass die Partikel kleiner als 50 nm groß sind.
Aus den TEM-Aufnahmen und der spezifischen Oberfläche wurde eine mittlere Korngröße von ca. 20 nm ermittelt.

Das mittels der Feinstvermahlung erhaltene Glaspulver weist eine Korngröße von 10 bis 50 nm auf. Bevorzugt beträgt die Korngröße ca. 20 bis 40 nm.

Die so hergestellten erfindungsgemäßen Dentalgläser können als Glasfüller für Dentalkomposite eingesetzt werden. Hierfür werden zunächst Haftvermittler mittels Silanisierens aufgebracht. Anschließend werden Stabilisatoren und Inhibitoren zugesetzt. Sodann erfolgt der Zusatz von anorganischen Füllern (Aerosil), YF₃, Farbstoffen/Pigmenten (z.B. Eisenoxid). Schließlich erfolgt die Einarbeitung in eine Monomermischung mit einem Polymerisationsinitiator, z.B. Campherchinon. Abschließend erfolgt sodann die Aushärtung.

Im Folgenden wird die Erfindung unter Bezugnahme auf die Beispiele näher erläutert:

### 1. Herstellung der Dentalgläser

Die Gläser wurden aus reinen Rohstoffen wie oben beschrieben bei Temperaturen zwischen 1600 und 1670°C erschmolzen und 1,5 Stunden homogenisiert.

Die Gläser wurden zur Erzeugung als Füllstoff als Glasfritte hergestellt. Das bedeutet, dass die Glasschmelze schnell abgeschreckt wird, so dass der hochgradig ungeordnete Zustand der Schmelze durch Unterkühlung eingefroren wird (eingefrorene unterkühlte Schmelze). Damit war es generell möglich, bei den erfindungsgemäßen MgOhaltigen Gläsern, die Phasentrennung zu umgehen und ein transparentes Glas in Pulverform zu erhalten.

Die Vorzerkleinerung der Glasfritte erfolgte je nach Anforderung mittels Strahl- oder Kugelmühle bis zu einer erreichten mittleren Korngröße von 5 bis 30 µm. Die Aufmahlung bis zur gewünschten Kornfeinheit (d₅₀ von ca. 1,5 µm nach 30 Minuten bzw. d₅₀ von ca. 0,7 µm nach 150 Minuten) erfolgte durch einen Nassvermahlung in einer Polyurethan- oder Keramikauskleidenden Ringspalt-Kugelmühle. Als Dispergiermittel wird deionisiertes Wasser eingesetzt bei einem Feststoffgehalt von 5 bis 50 Gew.-%. Als Mahlkörper wurden yttriumstabilisierte ZrO₂-Mahlkörper, Al₂O₃ oder auch Glasmahlkörper der gleichen Zusammensetzung zur Autozerkleinerung eingesetzt.

Nach einem ersten Zerkleinerungsschritt mit einem Feststoffgehalt von 33 Gew.-% und einem anschließenden zweiten Zerkleinerungsschnitt mit einem Feststoffgehalt von 10 Gew.-% wurde die spezifische Oberfläche des Mahlgutes gemessen. Nach 480 Minuten Mahldauer bei 33 Gew.-% wurde eine spezifische Oberfläche von 55,5 m²/g und nach einer Verringerung des Feststoffgehaltes auf 10 Gew.-% und weiteren 480 Minuten Mahldauer wurde eine spezifische Oberfläche von 106,9 m²/g erreicht.

Die Brechzahl wurde an Glaspulver mittels Becke-Methode ermittelt. Verwendet wurden Cargille Refractive Index Liquids als Vergleichslösungen unter einem Polarisationsmikroskop Metalloplan, Fa. Leitz.

Die Röntgenopazität der Dentalgläser wurde nach ISO 4049 (Radio-Opazität von dentalen Kompositmaterialien) an Massivproben mit 1 mm Dicke bestimmt. (Gerät: Oralix DC von Gentix und mit Prepress RP115 von Shamrock evaluiert). Als Referenz für das Maß der Röntgenopazität wird eine Aluminiumstandardspindel (Al 99.5%) von 1 bis 5 mm Dicke verwendet.

Die chemische Beständigkeit gegen NaF-Lösung wurde nach folgendem Verfahren ermittelt:
Für den qualitativen Korrosionstest wurden 3 Probekörper benötigt. Zwei Probekörper wurden in der NaF-Lösung untersucht, der Dritte diente als Referenzprobe. Zur Herstellung der Plättchen-Prüfkörper wurde die Monomermischung vorgelegt, der silanisierte Glasfüller (50 Masse%) mit einem Kunststoffspatel eingearbeitet und per Hand homogenisiert. Die Monomer-Füllstoffmischung wurde blasenfrei in die Prüfkörperform aus Stahl (rund, Durchmesser 15 mm, 1,5 mm Dicke) gestrichen und heißgehärtet. Die Aushärtung des Monomers findet unter Druck, Hitze und im Wasserbad im Ivomat IP® 3 (Ivoclar Vivadent AG) statt (10 Minuten bei 120°C und 6 bar).

Anschließend wurden die Probekörper mit 1000er SiC-Papier vorgeschliffen und auf Hochglanz mit einer 6µm-Diamant-Suspension beidseitig poliert.

Für den NaF-Test wurden zwei Probekörper in 100 ml- 0,001 %ige NaF-Lösung im Rückflusskühler 16 Stunden gekocht. Die Auswertung erfolgt qualitativ (visuell) hinsichtlich Farb- und Transparenzänderungen (Vergleich der Eintrübung zur Referenzprobe) und per REM-Aufnahme. Im REM-Bild wurde das Auftreten von möglichen Spalten zwischen den Füllstoffpartikeln und der umgebenden Monomermatrix untersucht. Sind die Füllstoffpartikel durch den Säuretest nicht chemisch angegriffen worden und somit resistent, so bleibt die Einbettung der Partikel in die Kompositmatrix intakt. Es treten keine Risse und Spalten auf. Die Transparenz des Komposit-Probekörpers ändert sich nicht.

### 2. Ausführungsbeispiele

Die folgende Tabelle umfasst Ausführungsbeispiele der erfindungsgemäßen Gläser in Form ihrer Zusammensetzungen und wichtigsten Eigenschaften.

### Angaben in Masse%

| | 1 | 2 | 3 | 4 | 5 | 6 |
|---|---|---|---|---|---|---|
| SiO₂ | 58,00 | 58,00 | 54,00 | 58,00 | 63,00 | 58,00 |
| CaO | 2,00 | 2,00 | 9,00 | 2,00 | | |
| Al₂O₃ | 14,00 | 14,00 | 10,00 | 14,00 | 12,40 | 14,00 |
| MgO | 6,93 | 7,93 | 8,00 | 6,43 | 10,40 | 6,10 |
| ZrO₂ | 2,00 | 2,00 | | 2,00 | 3,90 | 0,40 |
| ZnO | 2,50 | 2,50 | 1,00 | | | |
| La₂O₃ | 9,57 | 1,57 | 7,00 | 1,57 | 8,20 | 1,50 |
| WO₃ | 2,00 | 10,00 | 5,00 | 10,00 | 2,00 | 10,00 |
| B₂O₃ | 3,00 | 2,00 | 6,00 | 6,00 | 0,10 | 10,00 |
| | | | | | | |
| Summe | 100,00 | 100,00 | 100,00 | 100,00 | 100,00 | 100,00 |
| | | | | | | |
| Schmelze | 1650 / 75 | 1800 / 80 | 1650 / 90 | 1650 /90 | 1650 / 75 | 1650 / 90 |
| (°C/min.) | 1670 / 75 | 1850 / 80 | | | 1670 / 75 | |
| Farbe (Glas) | Leicht Gelblich | Leicht Gelblich | Farblos | Farblos | Leicht Gelblich | farblos |
| Transparenz | sehr gut | sehr gut | sehr gut | gut | gut | gut |
| Brechungsindex (Becke) | 1,546 | 1,534 | 1,548 | 1,528 | 1,548 | 1,512 |
| Röntgenopazität (%Al) | 281 | 352 | 274 | 284 | 276 | 263 |
| Beständigkeit gegen NaF | sehr gut | sehr gut | Sehr gut | Sehr gut | sehr gut | genügend, leichte Eintrübung |

### 3. Herstellung eines Füllungskomposites auf der Basis von röntgenopakten Füllern

Entsprechend der nachfolgend aufgeführten Tabelle wurde ein Füllungskomposit auf der Basis einer Methacrylatmischung unter Einbeziehung der röntgenopaken Füller mittels eines Laborkneters LPM 0,1 (Fa. Linden, Marienheide) hergestellt.

Die gemahlenen Füllstoffe wurden vorab gemäß folgender Rezeptur mit Silan oberflächenbehandelt (Angaben in Gew.-%):

| | |
|---|---|
| 90 | % Füller |
| 9 | % γ-Methacryl-oxypropyl-trimethoxy-Silan |
| 1 | % Wasser |

Die Inhaltstoffe wurden zusammengegeben und in einem Turbola-Mischer 24 Stunden homogenisiert. Anschließend wurde der Füller bei 50°C über einen Zeitraum von 4 Tagen getrocknet.

### Monomerzusammensetzung:

Um mit Füllern in einem Brechungsindexbereich von 1,50 bis 1,549 hochtransparente Composites herstellen zu können; ist es notwendig, den Brechungsindex der Monomermischung an den Füller anzupassen. Aufgrund des stark unterschiedlichen Brechungsindexes von TEGDMA und Bis-GMA ist dies möglich. Nachfolgende Tabelle zeigt den Brechungsindex der Monomermatrix in Abhängigkeit des Mischungsverhältnisses von TEGDMA und Bis-GMA (Angaben in Gew.-%)

| Bis-GMA | TEGDMA | Brechungsindex |
|---|---|---|
| 85 | 15 | 1,5370 |
| 70 | 30 | 1,5233 |
| 41 | 59 | 1,4970 |
| 28 | 74 | 1,4849 |
| 10 | 90 | 1,4703 |

Bei der Aushärtung der Monomermatrix zu einem Polymer erfolgt eine Zunahme der Dichte und damit eine Erhöhung des Brechungsindex um ca. 0,025. Damit wird mit diesen Monomermischungen der im Patent beanspruchte Brechungsindexbereich der Gläser abgedeckt. Die meisten in der Dentalindustrie verwendeten Monomere sind in einem Brechungsindexbereich von 1,47 bis 1,53, so dass die erfindungsgemäßen Füllstoffe auch für andere Monomeren verwendet werden können.

Die Monomere werden initiiert mit Campherchinon (0,25%), p-N,N-Dimethylaminobenzoesäureethylester (0,50%) und Lucirin® TPO (0,07%, BASF).

Mit den lichthärtend initiierten Monomermischungen und den erfindungsgemäßen Füllern wurden Composites hergestellt, wobei die Füller zu 70 Gew.-% eingesetzt wurden. Zur Messung der Röntgenopazität wurde das Composite 2 mal 3 Minuten mit einer dentalen Lichtquelle Spectramat (Vivadent) bestrahlt und damit ausgehärtet.

## Patentansprüche

1. Dentalglas umfassend 50 bis 70 Ma.-% SiO₂,
5 bis 18 Ma.-% Al₂O₃,
6,1 bis 30 Ma.-% MgO,
1 bis 15 Ma.-% La₂O₃,
1 bis 15 Ma.-% WO₃,
0,1 bis 8 Ma.-% ZrO₂,
und gegebenenfalls weitere Oxide mit Ausnahme der Oxide von Sr, Ba oder Alkalimetalle, wobei die angegebenen Anteile sich insgesamt auf 100 Ma.-% addieren.

2. Dentalglas nach Anspruch 1 **dadurch gekennzeichnet, daß** die Summe von La₂O₃ + WO₃ 8 bis 20 Ma.-% beträgt.

3. Dentalglas nach Anspruch 1 **dadurch gekennzeichnet, daß** die Summe von Al₂O₃ + La₂O₃ + WO₃ 16 bis 38 Ma.-% beträgt.

4. Dentalglas nach Anspruch 1 **dadurch gekennzeichnet, dass** es
50 bis 70 Ma.-% SiO₂,
9 bis 15 Ma.-% Al₂O₃,
6,1 bis 30 Ma.-% MgO,
1 bis 18 Ma.-% La₂O₃ + Y₂O₃
1 bis 12 WO₃ und
0,1 bis 8 ZrO₂, enthält,
wobei die Summe aller Komponenten sich auf 100% addiert

5. Dentalglas nach Anspruch 4 **dadurch gekennzeichnet, daß** die Summe aus La₂O₃ + Al₂O₃ + Y₂O₃ + ZrO₂ 16 bis 35 Ma.-% beträgt.

6. Dentalglas nach Anspruch 1 **dadurch gekennzeichnet, dass** es 8 bis 20 Ma.-% MgO enthält.

7. Dentalglas nach Anspruch 1 **dadurch gekennzeichnet, dass** es 0 bis 15 Ma.-% Oxide von weiteren zweiwertigen Metallen, ausgenommen Sr und Ba, enthält.

8. Dentalglas nach Anspruch 7 **dadurch gekennzeichnet, dass** es CaO und ZnO enthält.

9. Dentalglas nach Anspruch 1 **dadurch gekennzeichnet, dass** es 0 bis 20 Ma.-% Oxide von weiteren dreiwertigen und/oder vierwertigen Metallen enthält.

10. Dentalglas nach Anspruch 9 **dadurch gekennzeichnet, dass** es B₂O₃ und/oder TiO₂ enthält.

11. Dentalglas nach Anspruch 1 **dadurch gekennzeichnet, dass** es 0 bis 4 Ma.-% Ag₂O enthält.

12. Dentalglas nach Anspruch 1 **dadurch gekennzeichnet, dass** es 0 bis 2 Ma.-% Halogenide und/oder Sulfate enthält.

13. Verfahren zur Herstellung von Dentalgläsern **dadurch gekennzeichnet, dass**
a) eine Glasschmelze in der Zusammensetzung nach einem der Ansprüche 1 bis 12 hergestellt,
b) durch Abkühlen der Schmelze eine Glasfritte hergestellt,
c) die Glasfritte vorzerkleinert wird und
d) eine Feinmahlung zu Pulver durchgeführt wird.

14. Verfahren nach Anspruch 13 **dadurch gekennzeichnet, dass** die Glasfritte durch Einfrieren der Schmelze hergestellt wird.

15. Verfahren nach Anspruch 13 **dadurch gekennzeichnet, dass** die Vorzerkleinerung der Glasfritte auf eine Größe von 5 bis 30 µm erfolgt.

16. Verfahren nach Anspruch 13 **dadurch gekennzeichnet, dass** die Feinmahlung mittels einer Nassmahlung durchgeführt wird.

17. Verfahren nach Anspruch 16 **dadurch gekennzeichnet, dass** die Feinmahlung in einer Suspension mit einem Feststoffgehalt von 5 bis 50 Gew.-% durchgeführt wird.

18. Verfahren nach Anspruch 16 **dadurch gekennzeichnet, dass** die Feinmahlung auf eine Korngröße von 20 bis 50 nm erfolgt.

19. Verwendung des Dentalglases nach einem der Ansprüche 1 bis 12 als Glasfüller für Dentalkomposite.

## Claims

1. Dental glass containing 50 to 70 wt.% of SiO₂,
5 to 18 wt.% of Al₂O₃,
6.1 to 30 wt.% of MgO,
1 to 15 wt.% of La₂O₃,
1 to 15 wt.% of WO₃,
0.1 to 8 wt.% of ZrO₂,
and optionally further oxides except for the oxides of Sr, Ba or alkali metals, wherein the stated quantities in total add up to 100 wt.%.

2. Dental glass according to Claim 1, **characterized in that** the sum of La₂O₃ + WO₃ is 8 to 20 wt.%.

3. Dental glass according to Claim 1, **characterized in that** the sum of Al₂O₃ + La₂O₃ + WO₃ is 16 to 38 wt.%.

4. Dental glass according to Claim 1, **characterized in that** it contains
50 to 70 wt.% of SiO₂,
9 to 15 wt.% of Al₂O₃,
6.1 to 30 wt.% of MgO,
1 to 18 wt.% of La₂O₃ + Y₂O₃,
1 to 12 of WO₃, and
0.1 to 8 of ZrO₂,
wherein the sum of all components adds up to 100 wt.%.

5. Dental glass according to Claim 4, **characterized in that** the sum of La₂O₃ + Al₂O₃ + Y₂O₃ + ZrO₂ is 16 to 35 wt.%.

6. Dental glass according to Claim 1, **characterized in that** it contains 8 to 20 wt.% of MgO.

7. Dental glass according to Claim 1, **characterized in that** it contains 0 to 15 wt.% of oxides of other divalent metals, except for Sr and Ba.

8. Dental glass according to Claim 7, **characterized in that** it contains CaO and ZnO.

9. Dental glass according to Claim 1, **characterized in that** it contains 0 to 20 wt.% of oxides of other trivalent and/or tetravalent metals.

10. Dental glass according to Claim 9, **characterized in that** it contains B₂O₃ and/or TiO₂.

11. Dental glass according to Claim 1, **characterized in that** it contains 0 to 4 wt.% of Ag₂O.

12. Dental glass according to Claim 1, **characterized in that** it contains from 0 to 2 wt.% of halides and/or sulfates.

13. Method for the production of dental glasses, **characterized in that**
a) a glass melt is produced in the composition according to one of Claims 1 to 12,
b) a glass frit is produced by cooling of the melt,
c) the glass frit is pre-pulverized and
d) a fine milling to powder is performed.

14. Method according to Claim 13, **characterized in that** the glass frit is produced by freezing of the melt.

15. Method according to Claim 13, **characterized in that** the prepulverization of the glass frit is effected to a size of 5 to 30 µm.

16. Method according to Claim 13, **characterized in that** the fine milling is effected by means of a wet milling.

17. Method according to Claim 16, **characterized in that** the fine milling is effected in a suspension with a solids content of 5 to 50 wt.%.

18. Method according to Claim 16, **characterized in that** the fine milling is effected to a particle size of 20 to 50 nm.

19. Use of the dental glass according to one of Claims 1 to 12 as a glass filler for dental composites.

## Revendications

1. Verre dentaire, comprenant 50 à 70 % en masse de SiO₂, 5 à 18 % en masse d'Al₂O₃, 6, 1 à 30 % en masse de MgO, 1 à 15 % en masse de La₂O₃, 1 à 15 % en masse de WO₃, 0,1 à 8 % en masse de ZrO₂, et éventuellement d'autres oxydes, à l'exception des oxydes de Sr, de Ba ou de métaux alcalins, la somme des pourcentages indiqués faisant 100 % en masse.

2. Verre dentaire selon la revendication 1, **caractérisé en ce que** la somme de La₂O₃ et de WO₃ est de 8 à 20 % en masse.

3. Verre dentaire selon la revendication 1, **caractérisé en ce que** la somme de Al₂O₃, de La₂O₃ et de WO₃ est de 16 à 38 % en masse.

4. Verre dentaire selon la revendication 1, **caractérisé en ce qu'**il contient 50 à 70 % en masse de SiO₂, 9 à 15 % en masse d'Al₂O₃, 6, 1 à 30 % en masse de MgO, 1 à 18 % en masse de La₂O₃ + Y₂O₃, 1 à 12 WO₃ et 0,1 à 8 ZrO₂, la somme de l'ensemble des composants faisant 100 %.

5. Verre dentaire selon la revendication 4, **caractérisé en ce que** la somme de La₂O₃, d'Al₂O₃, d'Y₂O₃ et de ZrO₂ est de 16 à 35 % en masse.

6. Verre dentaire selon la revendication 1, **caractérisé en ce qu'**il contient 8 à 20 % en masse de MgO.

7. Verre dentaire selon la revendication 1, **caractérisé en ce qu'**il contient 0 à 15 % en masse d'oxydes d'autres métaux divalents, à l'exception de Sr et de Ba.

8. Verre dentaire selon la revendication 7, **caractérisé en ce qu'**il contient du CaO et du ZnO.

9. Verre dentaire selon la revendication 1, **caractérisé en ce qu'**il contient 0 à 20 % en masse d'oxydes d'autres métaux trivalents et/ou tétravalents.

10. Verre dentaire selon la revendication 9, **caractérisé en ce qu'**il contient du B₂O₃ et/ou du TiO₂.

11. Verre dentaire selon la revendication 1, **caractérisé en ce qu'**il contient 0 à 4 % en masse de Ag₂O.

12. Verre dentaire selon la revendication 1, **caractérisé en ce qu'**il contient 0 à 2 % en masse d'halogénures et/ou de sulfates.

13. Procédé de fabrication de verres dentaires, **caractérisé en ce que**
a) on fabrique un bain de verre, ayant la composition selon l'une des revendications 1 à 12,
b) par refroidissement du bain, on fabrique une fritte de verre,
c) on procède à un pré-broyage de la fritte de verre, et
d) on procède à un broyage fin pour obtenir une poudre.

14. Procédé selon la revendication 13, **caractérisé en ce que** la fritte de verre est fabriquée par figeage du bain.

15. Procédé selon la revendication 13, **caractérisé en ce que** le pré-broyage de la fritte de verre est effectué jusqu'à une granulométrie de 5 à 30 µm.

16. Procédé selon la revendication 13, **caractérisé en ce que** le broyage fin est effectué à l'aide d'un broyage par voie humide.

17. Procédé selon la revendication 16, **caractérisé en ce que** le broyage fin est effectué dans une suspension ayant une teneur en extrait sec de 5 à 50 % en poids.

18. Procédé selon la revendication 16, **caractérisé en ce que** le broyage fin est effectué jusqu'à une granulométrie de 20 à 50 nm.

19. Utilisation du verre dentaire selon l'une des revendications 1 à 12 en tant que charge de verre pour composites dentaires.
